# EUROPEAN PATENT APPLICATION

(11) **EP 1 759 658 A1**
(43) Date of publication of application: **07.03.2007**
(21) Application number: 06120113.3
(22) Date of filing: 05.09.2006
(51) Int. Cl.: A61C 8/00, A61B 17/16, A61B 17/17

(54) **Set of surgical instruments for dental implants**

(30) Priority: 06.09.2005 IT BO20050545
(71) Applicant: Franchini, Federico, 61011 Gabicce Mare (IT)
(72) Inventor: Franchini, Federico, 61011 Gabicce Mare (IT)
(74) Representative: Jorio, Paolo

(57) **Abstract**

A set (1) of surgical instruments for dental implants presenting a calibrated bushing (2), at least one calibrated endmill (3; 4), an implant screw (5), an inserter (6) for implant screw (5), and a copy (9) of the implant screw (5); the endmill (3; 4) and the inserter (8) present respective abutting edges (17; 22), each of which presents a larger diameter with respect to the internal diameter of the calibrated bushing (2) to define a fixed and determined maximum insertion position of the endmill (3; 4) or of the inserter (8) within the calibrated bushing itself (2); on the external surface of the inserter (8) is present at least one reference notch (23) for indicating the angular position of the inserter itself (8).

## Description

The present invention relates to a set of surgical instruments for dental implants.

In order to rehabilitate missing teeth consequent to periodontal condition or other by means of an osseointegrated implant, modern oral osseointegrated implant techniques envisage to prepare the implant site by drilling the dental arch using a rotating endmill fitted on a controlled speed handpiece. The osseointegrated implant and in particular the implant screw is then inserted in the implant site at a controlled torque by means of the previously used controlled speed handpiece.

The most complex step of the implant procedure described above is the drilling of the jawbone in order to achieve the implant site inside the jawbone itself, because also minor positioning errors, related to inclination and depth of the implant site, may compromise the final result of the implant, both in aesthetic and functional terms (overbite, chewing, speaking). Furthermore, during drilling of the jawbone, one must carefully avoid compromising the nerve and vascular structures present in the jawbone.

The use of a surgical template, which is coupled to the jawbone before drilling the jawbone, was introduced in an attempt to assist the dentist in preparing the implant site. The surgical template presents a through guide hole, which is positioned so as to be exactly coaxial with the hole to be drilled in the jawbone; therefore, once the surgical template is positioned on the jawbone, the dentist only needs to insert the tip of the calibrated endmill within the guide hole to accurately prepare the implant site in the jawbone. Preferably, the guide hole of the surgical template is internally lined by a calibrated bushing, which is provided by the manufacturer of the dental implant and generally presents an internal diameter equal to the external diameter of the calibrated surgical endmill.

In general, the precision of the hole drilled in the jawbone is higher proportionally to the accuracy with which the surgical template is made and in particular is proportional to the precision with which the guide hole in the surgical template is made. Until a few years ago, guide templates for inserting osseointegrated implants in jaws were made entirely by hand, with the help of plaster cast model of the patent's dental arches. In order to increase the precision of the guide templates for inserting osseointegrated implants in jawbones, it has recently been proposed to make such guide templates by means of CAD/CAM techniques which are based on a three-dimensional virtual reproduction of the skull obtained by means of a maxillary CT (Computerised Tomography) scan. An example of a CAD/CAM technique for making a surgical template for inserting osseointegrated implant in jawbones is provided by patent application US2004078212A1.

After inserting the osseointegrated implant, and in particular the implant screw, in the implant site, the exact axial position (height) and the exact angular orientation of the head of the implant screw must be determined (for example by making a cast). After detecting the exact axial position (height) and extract angular position of the implant screw head, a plaster model of the jawbone is reproduced so as to use the plaster model of the jawbone for making the prosthetics to be fixed to the osseointegrated implant.

In other words, after inserting the osseointegrated implant, the dentist must determine the exact axial (height) and exact angular position of the head of the implant screw and then must temporarily stitch the gum to cover the head of the implant screw. At this point, the dentist communicates the exact axial position (height) and exact angular orientation of the head of the implant screw to a dental mechanics laboratory, where the plaster model of the jawbone is used to made the prosthetics to be fixed to the osseointegrated implant. Finally, the dentist must intervene at a second time in the patent's mouth to remove the previously applied stitches and to fix the prosthetics made by the dental mechanics laboratory to the osseointegrated implant.

It is obvious that the need to determine the exact axial position (height) and the exact angular orientation of the implant screw head in the jawbone causes lengthening of time and above all forces the dentist to intervene on the patient in two subsequent movements with evident inconvenience for the patient.

It is the object of the present invention to provide a set of surgical instruments for dental implants, which is free from the drawbacks described above, easy and cost-effective to implement and allows to position an implant screw at a previously determined axial position (height) and angular orientation.

According to the present invention, a set of surgical instruments for dental implants as recited in the attached claims is provided.

The present invention will now be described with reference to the accompanying drawings illustrating a non-limitative embodiment, in which:
- figure 1 shows a perspective view of a set of surgical instruments for dental implants made according to the present invention;
- figure 2 shows a different perspective view of the set of surgical instruments in figure 1;
- figure 3 shows a side sectional view of the set of surgical instruments in figure 1;
- figure 4 shows a perspective and schematic view of a jawbone plaster model of a patient provided with two implant screws;
- figure 5 shows a perspective and schematic view of the plaster model in figure 4, in which one implant screw is engaged by one of the surgical instruments in figure 1;
- figure 6 shows a perspective and schematic view of the plaster model in figure 4 coupled to a surgical template and in which one implant screw is engaged by one of the surgical instruments in figure 1;
- figures 7 and 8 show a perspective and schematic view of an implant site by means of one of the surgical instruments in figure 1 and with the assistance of a surgical template; and
- figures 9, 10 and 11 show schematically and in side section a sequence of operations which are performed using the surgical instrument set in figure 1 for installing a dental implant.

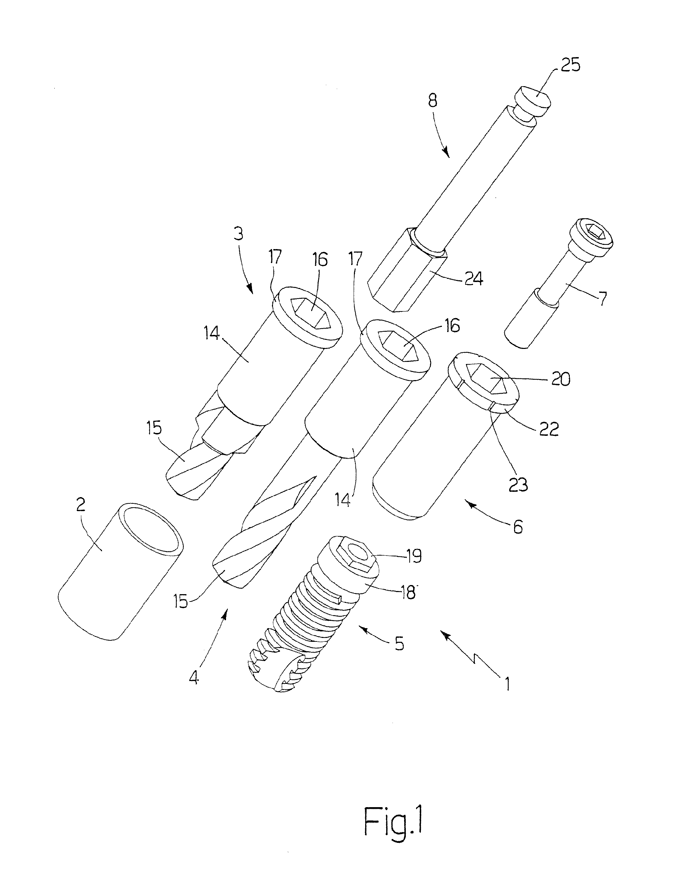

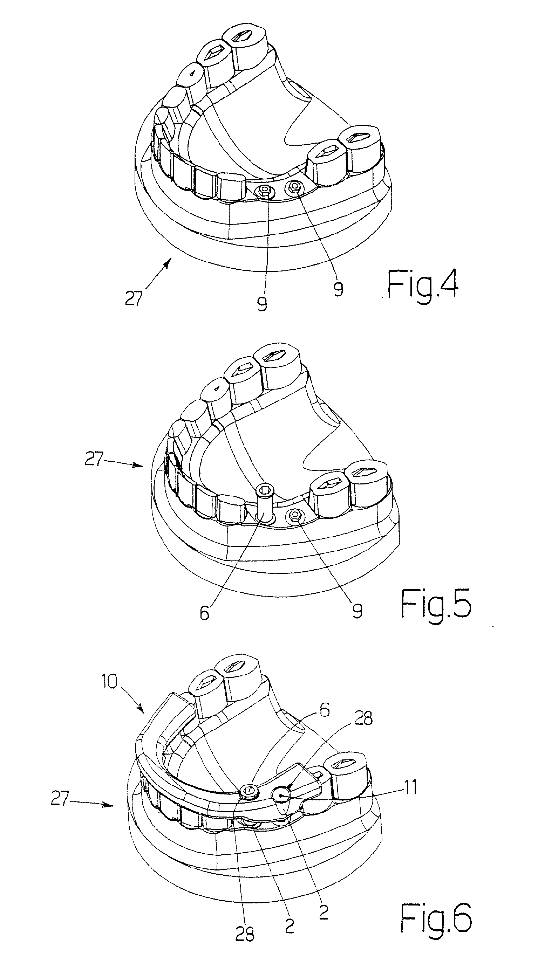

In figure 1, number 1 indicates a set of surgical instruments for dental implants. Set 1 of surgical instruments comprises a calibrated bushing 2, a initial calibrated endmill 3, a final calibrated endmill 4, an implant screw 5, an inserter 6 for implant screw 5, a retainer screw 7 for implant screw 5 and a feeder 8 for endmills 3 and 4 and for inserter 6. Furthermore, set 1 of surgical instruments also comprises a copy 9 of implant screw 5 (partially shown in figures 4 and 5), which is essentially identical to implant screw 5 and is adapted to be inserted in an implant site made in a plaster model of the patient's jawbone.

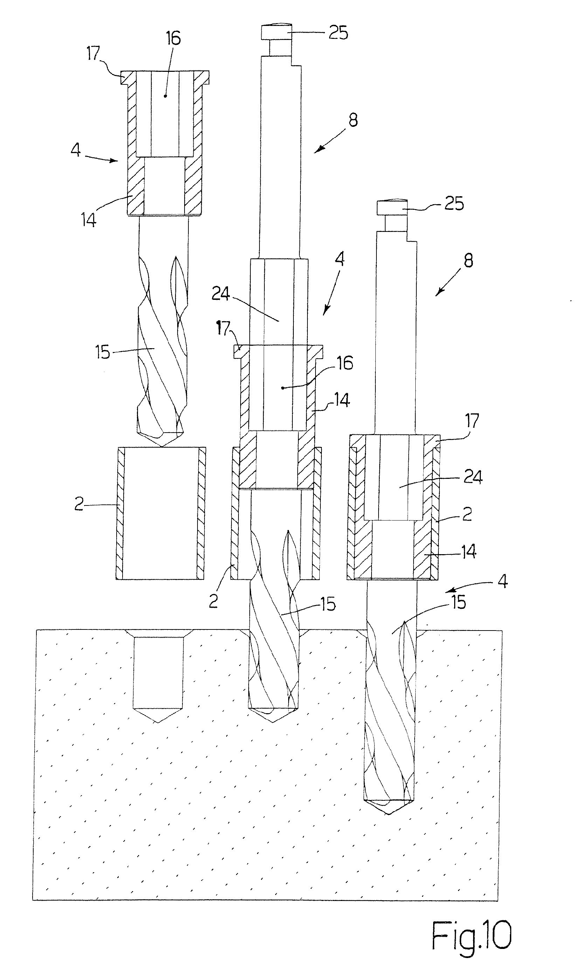

Calibrated bushing 2 presents a cylindrical tubular shape and is used to internally line a guide hole of a surgical template. Figures 6, 7 and 8 show a surgical template 10, which is coupled to the jawbone before drilling the jawbone and presents a pair of through guide holes 11, each of which is positioned so as to be exactly coaxial with the hole to be made through the jawbone for creating implant site 12 (shown in figure 10).

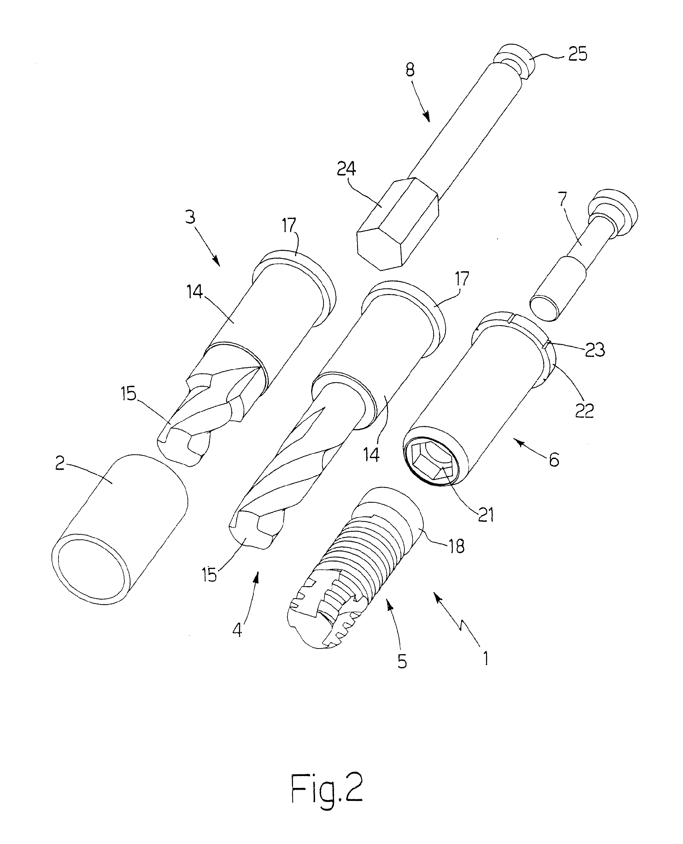

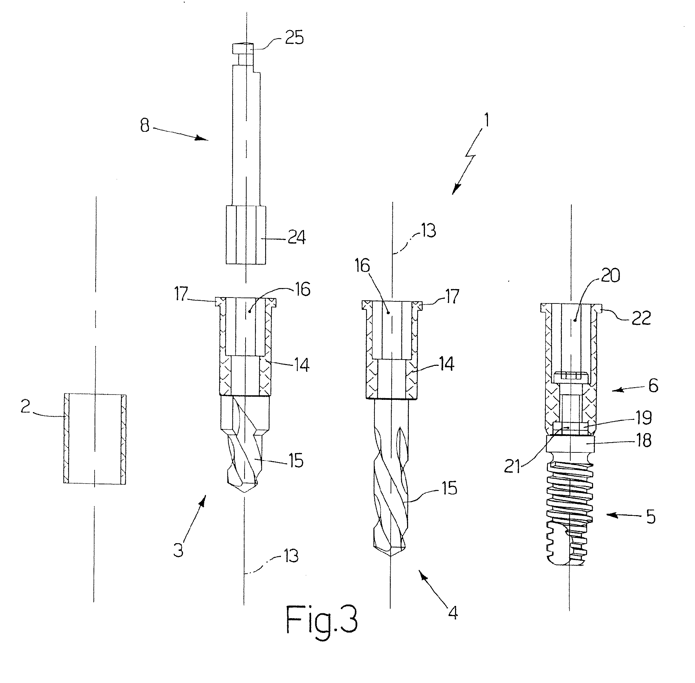

As shown in figures 1, 2 and 3, each endmill 3 or 4 presents a cylindrical symmetry around a central axis 13 and comprises a bushing 14 from which a cutting tip 15 extends. The external diameter of each cutting tip 15 is smaller than the internal diameter of the calibrated bushing 2, while the external diameter of each bushing 14 is essentially identical to the internal diameter of the calibrated bushing 2. Obviously, the shape of each tip 15 may differ according to the shape of the implant site 12 to be made (for example a cylindrical shape implant site 12 as shown in the attached figures, or a tapered shape implant site 12). Each bushing 14 presents a hexagonal blank hole 16 and an abutting edge 17, whose external diameter is larger than the internal diameter of the calibrated bushing 2 and consequently defines a fixed and determined maximum insertion position of the endmill 3 or 4 within the calibrated bushing 2 itself.

The difference between initial endmill 3 and final endmill 4 is essentially the length (i.e. the dimension along central axis 13) of cutting tip 15, because initial mill 3 is used to make a first drilling in the jawbone, while final endmill 4 is used to complete drilling of the jawbone. It is apparent that the total number of endmills 3 and 4 may differ from two according to the depth of implant site 12 to be prepared; for example set 1 of surgical instruments may comprise a single endmill or three or four endmills.

Implant screw 5 presents an upper wall 18, from which external hexagonal attachment 19, commercially known as "Branemark", protrudingly rises; obviously, the shape of attachment 19 may be different. Inserter 6 is cylindrical and presents a hexagonal blank hole 20 at an upper end and a hexagonal blank hole 21 at a lower end, which reproduces in negative the shape of attachment 19 of implant screw 5 to be coupled to attachment 19 itself.

At the upper end, inserter 6 presents an abutting edge 22, whose external diameter is larger than the internal diameter of the calibrated bushing 2 and consequently defines a fixed and determined maximum insertion position of inserter 6 within calibrated bushing 2 itself. On abutting edge 22 of inserter 6 there are six notches 23, which provide with a sufficient accuracy the angular position of inserter 6, and consequently of an implant screw 5 coupled to inserter 6. To better identify the angular position of inserter 6, notches 23 may be identified by means of an alphanumeric code (i.e. numbers or letters) or may be reciprocally differentiated by shape and/or dimensions. Obviously, the number of notches 23 may differ from six, for example four or eight notches 23 may be envisaged; furthermore, any other type of visible marker may be used instead of notches 23.

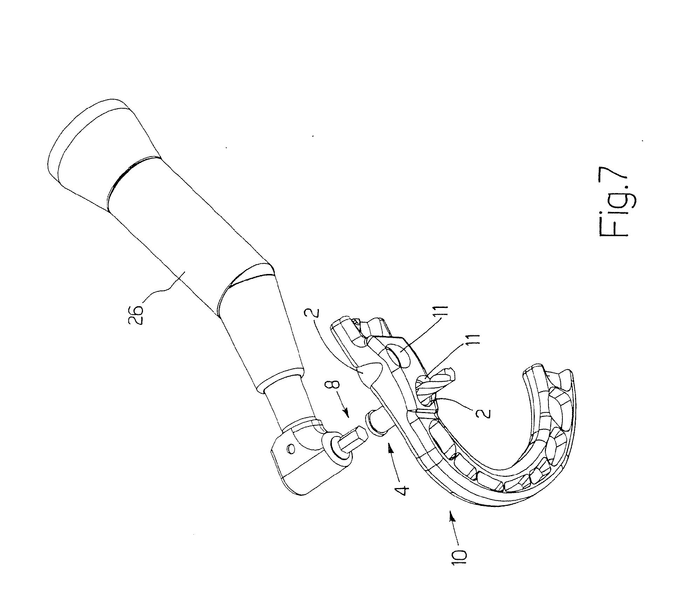

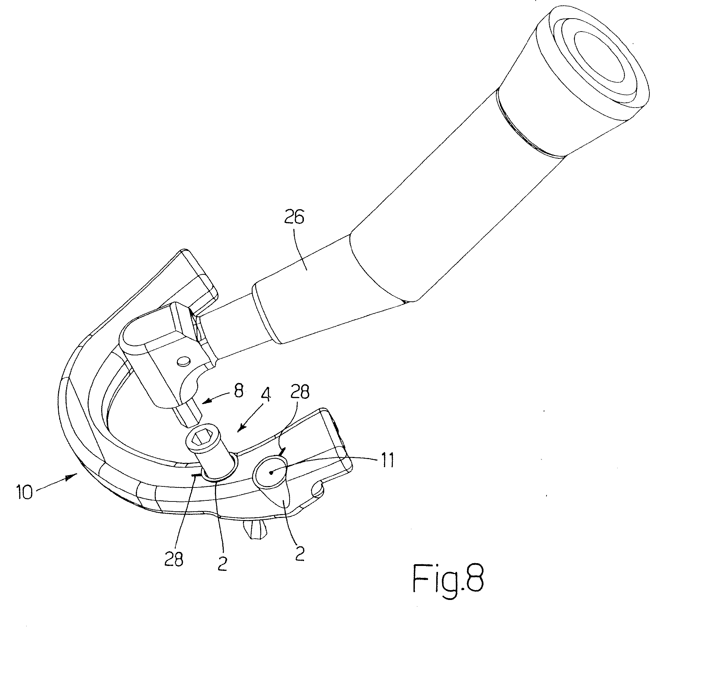

Feeder 8 is shaped as a cylindrical rod; at a lower end, feeder 8 presents a hexagonal coupling element 24, which is shaped and dimensioned so as to be coupled without clearance in hole 16 of each endmill 3 or 4 and in hole 20 of inserter 6. At an upper end, feeder 8 presents a coupling element 25, which is shaped and dimensioned to be mechanically coupled with a dental tool 26, in particular an adjustable speed contra-angle handpiece (shown in figures 7 and 8). In the attached figures, coupling element 24 is hexagonal; according to a different embodiment (not shown), coupling element 24 may have a different shape, for example, polygonal with rounded sides to allow misalignments of the dental tool 26 without forcing the positioning of the surgical template 10.

The methods of inserting an osseointegrated implant for the prosthetic restoration 2 of individual teeth using set 1 of surgical instruments is described below.

As shown in figures 4, 5 and 6, initially employing a standard, widely known method, a plaster model 27 of the patient's dental arches is made; in particular, a plaster model 27 of the lower dental arch and a plaster model 27 of the upper dental arch are made (only one of the two plaster models 27 is shown in figures 4, 5 and 6). Once completed, the plaster models 27 are fitted into a dental articulator (known and not shown), which reproduces the movements of the patent's mandible and consequently reproduces the correct intermaxillary relation between the two plaster models 27.

By using plaster models 27 and possibly also a three-dimensional virtual reproduction of the skull obtained by maxillary CT (Computerised Tomography) scan, the osseointegrated implant is designed, and in particular the position and the dimension of the two implant sites which will be prepared by drilling the jawbone by means of endmills 3 and 4 is determined. After defining the position and dimension of the two implant sites, surgical template 10 is made presenting two through guide holes 11, each of which is positioned so as to be exactly coaxial with the hole to be made in the jawbone to create implant seat 12. A calibrated bushing 2 is inserted and locked in a certain position by means of resin within each guide hole 11 of surgical template 10.

By using the known methods, surgical template 10 may be made entirely by hand with the help of plaster models 27, or using CAD/CAM techniques, for example using a numeric control robot (such as the handling robot known as "Kapto04").

By using endmills 3 and 4 and template 10, plaster model 27 of the jawbone which will receive the two implants is drilled to insert the two copies 9 of implant screw 5; subsequently, such plaster model 27 is used to made two prosthetics (not shown) which will be fixed to the implants.

At this point, surgical template 10 is coupled to plaster model 27 of the jawbone which will receive the two implants and through each calibrated bushing 2 is inserted inserter 6 to determine the exact angular position of the two copies 9 of implant screw 5; for this purpose, on the surgical template and in proximity of each calibrated bushing 2 is marked at least one reference notch 28 which generally matches with one of the notches 23 present on abutting edge 22 of inserter 6.

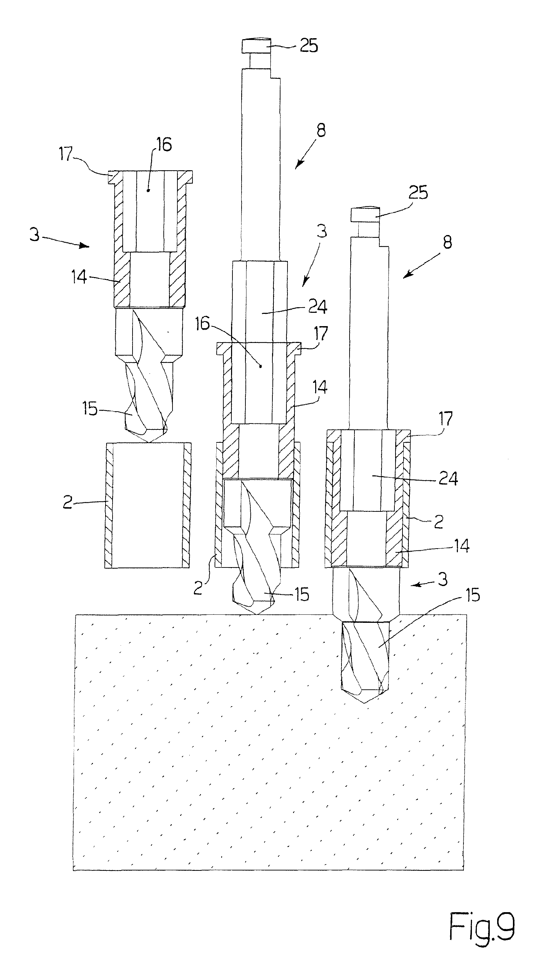

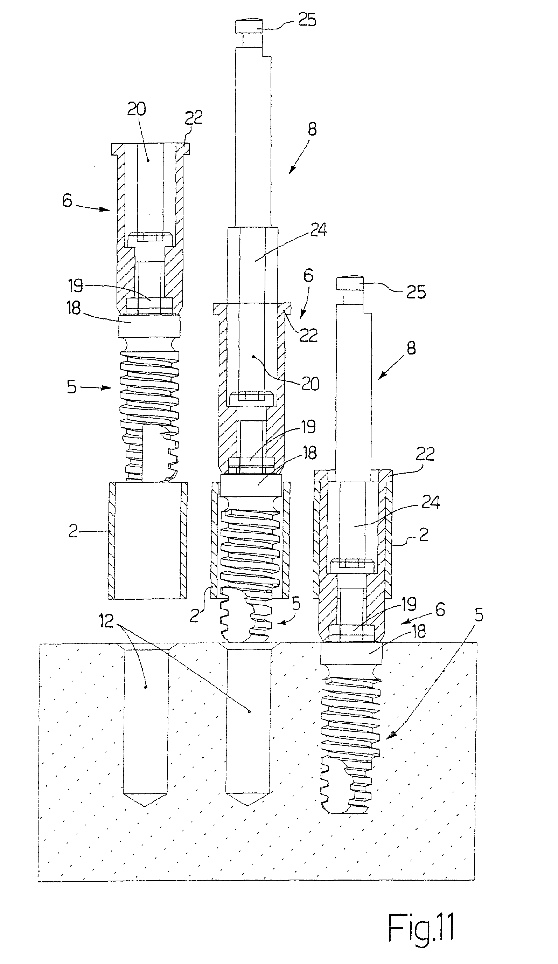

Now, the surgical procedure within the patient's mouth may start, the procedure consisting in three steps schematically shown in figures 9, 10 and 11. Three subsequent moments are illustrated for each step: supplantation of guide bushing 2, start of dental tool 26 use, and end of dental tool 26 use.

Initially, surgical template 10 is positioned on the patient's dental arch which will receive the implants and through each calibrated bushing 2 is inserted the initial endmill 3 coupled to dental tool 26 by means of feeder 8 for a first drilling of the jawbone (figure 9). As clearly shown in figure 9, tip 15 of endmill 3 initially touches the alveolar ridge of the jawbone only after bushing 14 of initial endmill 3 is already inserted within guide bushing 2 sufficiently to maintain axiality during perforation. Furthermore, the alveolar ridge is the hardest part to treat, and therefore it is appropriate for the engagement zone of coupling element 24 of feeder 8 to be as close to the alveolar ridge as possible.

Subsequently, through each calibrated bushing 2, final endmill 4 coupled to dental tool 26 by means of feeder 8 is inserted to complete drilling of the jawbone and consequently prepare implant site 12 (figure 10). By the way of example, figures 7 and 8 show a perspective and schematic view of the jawbone drilling procedure by means of final endmill 4. It is important to observe that the axial position of each implant site 12 is accurately determined by the cylindrical coupling between bushing 14 of endmills 3 and 4 and calibrated bushing 2, while the axial dimension (depth) of each implant site 12 is accurately determined by the abutment of abutting edge 17 of bushing 14 of endmills 3 and 4 against calibrated bushing 2. As clearly shown in figure 9, tip 15 of final endmill 4 is centred in the hole previously made by initial endmill 3 and in calibrated bushing 2 before starting to operate, ensuring the axial position of implant site 12.

Finally, through each calibrated bushing 2 an implant screw 5 coupled to inserter 6, coupled in turn coupled to dental tool 26 by means of feeder 8, is arranged to insert implant screw 5 itself within implant site 12. It is important to observe that the axial position of each implant screw 5 is accurately determined both by the position of implant site 12 and by the cylindrical coupling between inserter 6 and calibrated bushing 2; while the axial position (depth) of each implant screw 5 is accurately determined by the abutment of the abutting edge 22 of inserter 6 against calibrated bushing 2. Finally, the angular position of each implant screw 5 may reproduce with an extreme accuracy the angular position of copy 9 of implant screw 5 in plaster model 26 by using notches 23 present on abutting edge 22 of inserter 6 and the reference notch (not shown) present on surgical template 10 in proximity of calibrated bushing 2.

After inserting the two implant screws 5 in the two implant sites 12, the prosthetics may be mounted on implant screws 5 themselves, thus completing the rehabilitation intervention on missing teeth.

Set 1 of surgical tools described above presents several advantages, because it is simple and cost-effective to make, simple to use, and above all allows to position implant screw 5 according to a predetermined axial position (height) and angular orientation identical to those present in the plaster model of the jawbone. In this way, the exact axial position (height) and exact angular orientation of the head of implant screw 5 within the jawbone do not need to be determined and the prosthetics can be prepared by using the plaster model of the jawbone before inserting implant screw 5 in the jawbone. Therefore, the dentist may operate in the patient's mouth once only, inserting implant screw 5 first and then immediately after fastening the prosthetics.

Furthermore, the surgical tools, in particular endmills 3 and 4, present an overall limited axial dimension (height) thus facilitating their use in the patient's mouth and reducing the patient's discomfort; in this regard, is must be observed that endmills 3 and 4 must be inserted within guide holes 11 of surgical template 10 and therefore if their overall axial dimension were longer, their insertion in guide holes 11 of surgical template 10 would be difficult.

As previously mentioned, for each endmill 3 or 4, the external diameter of cutting tip 15 is smaller than the internal diameter of calibrated bushing 2, while the external diameter of bushing 14 is essentially identical to the internal diameter of calibrated bushing 2; consequently, each endmill 3 and 4 is guided along calibrated bushing 2 only by bushing 14 and the cutting tip 15 does not come into contact with the internal surface of the calibrated bushing 2. By avoiding the contact of cutting means, i.e. the sides of tip 15, against the internal surface of calibrated bushing 2, implant site 12 is protected from contamination of metallic dust which otherwise may be scraped from internal surface of the calibrated bushing 2 itself.

Finally, by constantly maintaining a safe axial guidance of endmills 3 and 4 and inserter 6 thanks to the cylindrical coupling between calibrated bushing 2 and bushings 14 and between calibrated bushing 2 and inserter 6, side stress in surgical template 10 is cancelled out or at least considerably reduced during the surgical step.

## Claims

1. A set (1) of surgical instruments for dental implants comprising:
a calibrated bushing (2), which presents a tubular cylindrical shape and which is adapted to be used for internally lining a guide hole (11) of a surgical template (10);
at least one endmill (3; 4) calibrated to perforate a jawbone and prepare an implant site (12);
an implant screw (5) adapted to be inserted in an implant site (12);
an inserter (6) for the implant screw (5) adapted to insert implant screw (5) within an implant site (12); and
a copy (9) of the implant screw (5), which is essentially identical to the implant screw (5) and adapted to be inserted in an implant site made in a plaster model (26) of the patient's jawbone;
the surgical instruments set (1) is **characterised in that**:
the endmill (3; 4) and the inserter (8) present respective abutting edges (17; 22), each of which has an external diameter larger than the internal diameter of the calibrated bushing (2) to define a fixed and determined maximum insertion position of the endmill (3; 4) or inserter (8) within the calibrated bushing (2) itself;
on the external surface of the inserter (8) is present at least one reference notch (23) to indicate the angular position of the inserter (8) itself.

2. A set (1) of surgical instruments, according to claim 1, and comprising a feeder (8), which is adapted to mechanically couple the endmill (3; 4) and the inserter (6) to a dental tool (26).

3. A set (1) of surgical instruments according to claim 2, wherein the feeder (8) has the shape of a cylindrical rod; at a lower end, the feeder (8) presents a first coupling element (24), which is shaped and dimensioned to couple with the endmill (3; 4) and with inserter (6); at an upper end, the feeder (8) presents a second coupling element (25), which is shaped and dimensioned to mechanically couple with the dental tool (26).

4. A set (1) of surgical instruments according to claim 3, wherein the first coupling element (24) is shaped so as to allow misalignments of the dental tool (26).

5. A set (1) of surgical instruments according to one of the claims from 1 and 4, and comprising an initial calibrated endmill (3) and a final calibrated endmill (4) of different length; the initial endmill (3) is adapted to make a first drilling of the jawbone, and the final endmill (4) is adapted to complete the drilling of the jawbone.

6. A set (1) of surgical instruments according to one of the claims from 1 to 5, wherein the endmill (3; 4) presents a cylindrical symmetry around a central axis (13) and comprises a bushing (14) from which a cutting tip (15) extends.

7. A set (1) of surgical instruments according to claim 6, wherein the external diameter of the cutting tip (15) is smaller than the internal diameter of the calibrated bushing (2), while the external diameter of the bushing (14) is essentially identical to the internal diameter of the calibrated bushing (2).

8. A set (1) of surgical instruments according to claim 6 or 7, and comprising a feeder (8), which is adapted to mechanically couple the endmill (3; 4) to a dental tool (26); the bushing (14) presents a blank hole (16) adapted to be engaged by the feeder (8).

9. A set (1) of surgical instruments according to claim 6, 7 or 8, wherein the abutting edge (17) of the endmill (3; 4) extends from an upper portion of the bushing (14).

10. A set (1) of surgical instruments according to one of the claims from 1 to 9, wherein the inserter (6) has a cylindrical shape and presents its abutting edge (22) at an upper end.

11. A set (1) of surgical instruments according to one of the claims from 1 to 10, wherein the first reference notch (23) is made on the abutting edge (22) of the inserter (6).

12. A set (1) of surgical instruments according to one of the claims from 1 to 11, wherein a set of reference notches (23) are present on the external surface of the inserter (8) for indicating the angular position of the inserter (8) itself.

13. A set (1) of surgical instruments according to claim 12, wherein the first reference notches (23) are identified by means of an alphanumeric code.

14. A set (1) of surgical instruments according to claim 12, wherein the first reference notches (23) are reciprocally differentiated by shape and/or dimensions.

15. A set (1) of surgical instruments according to one of the claims from 1 to 14, and comprising a surgical template (10) adapted to be coupled to a jawbone before drilling the jawbone itself and presenting at least one guide hole (11), which is positioned so as to be coaxial with a hole drilled in the jawbone to create an implant site (12) and accommodates the calibrated bushing (2); on the surgical template and in proximity of the calibrated bushing (2) is marked at least one second reference notch (28), which along with the first reference notch (23) indicates the angular position of the inserter (8) when the inserter (8) itself is inserted within the calibrated bushing (2).
